# EUROPEAN PATENT SPECIFICATION

(11) **EP 3 026 119 B1**
(45) Date of publication and mention of the grant of the patent: **18.09.2019**
(21) Application number: 15175155.9
(22) Date of filing: 04.01.2007
(51) Int. Cl.: C12Q 1/25, G01N 33/50, G01N 33/574

(54) **USE OF HE4 AND OTHER BIOCHEMICAL MARKERS FOR ASSESSMENT OF OVARIAN CANCERS**
VERWENDUNG VON HE4 UND ANDEREN BIOCHEMISCHEN MARKERN ZUR ANALYSE VON OVARIALKREBSERKRANKUNGEN
UTILISATION DE HE4 ET D'AUTRES MARQUEURS BIOCHIMIQUES DANS L'ÉVALUATION DE CANCERS DE L'OVAIRE

(30) Priority: 04.01.2006 US 756131 P
(43) Date of publication of application: 01.06.2016
(62) Divisional of application: 07717677.4
(73) Proprietor: Fujirebio America, Inc., Wilmington, DE 19808 (US)
(72) Inventor: MOORE, Richard, Cranston, RI 02921 (US); SOMERS, Elizabeth, Unionville, PA 19375 (US)
(74) Representative: HGF Limited

(56) References cited:
- EP-A- 1 846 768
- WO-A-03/021273
- WO-A-2006/089125
- US-A1- 2005 214 826
- ROSEN ET AL: "Potential markers that complement expression of CA125 in epithelial ovarian cancer", GYNECOLOGIC ONCOLOGY, ACADEMIC PRESS, LONDON, GB, vol. 99, no. 2, 1 November 2005 (2005-11-01), pages 267-277, XP005118976, ISSN: 0090-8258
- DRAPKIN R ET AL: "Human epididymis protein 4 (HE4) is a secreted glycoprotein that is overexpressed by serous and endometrioid ovarian carcinomas", CANCER RESEARCH, AMERICAN ASSOCIATION FOR CANCER RESEARCH, US, vol. 65, no. 6, 15 March 2005 (2005-03-15) , pages 2162-2169, XP002600809, ISSN: 0008-5472, DOI: 10.1158/0008-5472.CAN-04-3924
- HELLSTROM I ET AL: "The HE4 (WFDC2) protein is a biomarker for ovarian carcinoma", CANCER RESEARCH, AMERICAN ASSOCIATION FOR CANCER RESEARCH, US, vol. 63, no. 13, 1 July 2003 (2003-07-01), pages 3695-3700, XP002290876, ISSN: 0008-5472
- KIRCHHOFF C ET AL: "A major human epididymis-specific cDNA encodes a protein with sequence homology to extracellular proteinase inhibitors", BIOLOGY OF REPRODUCTION, NEW YORK, NY [U.A.] : ACADEM. PRESS, US, vol. 45, no. 2, 1 August 1991 (1991-08-01) , pages 350-357, XP002572895, ISSN: 0006-3363, DOI: 10.1095/BIOLREPROD45.2.350
- K. K. ZORN ET AL: "Gene Expression Profiles of Serous, Endometrioid, and Clear Cell Subtypes of Ovarian and Endometrial Cancer", CLINICAL CANCER RESEARCH, vol. 11, no. 18, 15 September 2005 (2005-09-15), pages 6422-6430, XP055050926, ISSN: 1078-0432, DOI: 10.1158/1078-0432.CCR-05-0508
- SHANTA V: "Perspectives in malignant ovarian tumours", INDIAN JOURNAL OF MEDICAL AND PAEDIATRIC ONCOLOGY, vol. 25, no. 3, 2004, pages 4-14, XP002514103,

## Description

### BACKGROUND OF THE DISCLOSURE

The disclosure relates generally to the field of diagnosis, grading, staging, and prognosis of cancer. More particularly, this disclosure relates to the field of ovarian cancers. Also, this disclosure relates to the field of ovarian cancer diagnosis, grading, staging, and prognosis involving expression of biological markers. More particularly, this disclosure relates to a method of assessing whether a patient is afflicted with stage I ovarian cancer, a method of differentiating whether a pelvic mass in a patient is benign or a stage I ovarian cancer, a method of assessing the response of a patient afflicted with stage I ovarian cancer to a treatment, and a method of assessing the likelihood that a patient will develop a stage I ovarian cancer.

Cancer includes a broad range of diseases, affecting approximately one in four individuals worldwide. The severity of the adverse impact of cancer is profound, influencing medical policy and procedure as well as society generally. Because a hallmark of many types of cancer is rapid and unregulated proliferation of malignant cells, an overarching problem in improving approaches to cancer is the need for early detection and diagnosis. Early detection is well regarded as the best means to reduce cancer mortality. As a result, numerous attempts have been made to develop accurate and reliable criteria for diagnosing the presence of a malignant condition. In particular, investigations have been directed to the use of serologically defined antigenic markers known as tumor associated antigens, which are either uniquely expressed by cancer cells or are present at markedly higher levels in subjects having a malignant condition.

However, due to the high heterogeneity of tumor associated antigen expression, for example the extreme diversity of carcinoma antigens, there is a need for additional tumor markers that are useful in cancer diagnosis. Many monoclonal antibodies reactive with carcinoma associated antigens are known. Such monoclonal antibodies bind to a variety of different carcinoma-associated antigens including glycoproteins, glycolipids, and mucins. Many such monoclonal antibodies recognize tumor-associated antigens that exhibit restricted expression on some, but not other, tumors originating in a given cell lineage or tissue type.

There are relatively few examples of tumor associated antigens that appear to be useful for identifying a particular type of malignancy. Monoclonal antibody B72.3, for example, specifically binds to a high molecular mass (>106 Da) tumor-associated mucin antigen that is selectively expressed on a number of different carcinomas, including most if not all ovarian carcinomas and an overwhelming majority of non-small cell lung carcinomas, colon carcinomas and breast carcinomas. Nevertheless, detection of cell-associated tumor markers such as the mucin antigen recognized by B72.3 following surgical resection of a tumor may be of limited usefulness for diagnostic screening, in which early detection of a malignant condition prior to accumulation of substantial tumor mass is preferred.

An alternative to the diagnosis of a particular type of cancer by screening surgically resected specimens for tumor associated antigens, where invasive surgery is usually indicated only after detection of an accumulated tumor mass, would be to provide compositions and methods for detecting such antigens in samples obtained from subjects by non-invasive or minimally invasive procedures. In ovarian, endometrial, and other carcinomas, for example, there are currently a number of soluble tumor associated antigens that are detectable in samples of readily obtained biological fluids such as serum or mucosal secretions. One such marker is CA125, a carcinoma-associated antigen that is also shed into the bloodstream, where it is detectable in serum (e.g. Bast et al., 1983 N. Eng. J. Med. 309:883; Lloyd et al., 1997 Int. J. Canc. 71:842). CA 125 levels in serum and other biological fluids have been measured along with levels of other markers, for example, carcinoembryonic antigen (CEA), squamous cell carcinoma antigen (SCC), tissue polypeptide specific antigen (TPS). Sialyl TN mucin (STN) and placental alkaline phosphatase (PLAP), in an effort to provide diagnostic and/or prognostic profiles of ovarian, endometrial, and other carcinomas (e.g., Sarandakou et al., 1997, Acta Oncol. 36:755; Sarandakou et al., 1998, Eur. J. Gynaecol. Oncol. 19:73; Meier et al., 1997, Anticanc. Res. 17(4B):2945; Kudoh et al., 1999, Gynecol. Obstet. Invest. 47:52; Ind et al., 1997, Br. J. Obstet. Gynaecol. 104:1024; Bell et al. 1998, Br. J. Obstet. Gynaecol. 105:1136; Cioffi et al., 1997, Tumori 83:594; Meier et al. 1997, Anticanc. Res. 17(4B):2949; Meier et al., 1997, Anticanc. Res. 17(4B):3019).

Elevated levels of serum CA125 alone or in combination with other known indicators, however, do not provide a definitive diagnosis of malignancy, or of a particular malignancy such as ovarian or endometrial carcinoma. For example, elevated CA125, CEA and SCC in vaginal fluid and serum correlate most strongly with inflammation in benign gynecological diseases, relative to cervical cancer and genital tract cancers (e.g., Moore et al., 1998 Infect. Dis. Obstet. Gynecol. 6: 182; Sarandakou et al., 1997 Acta Oncol. 36:755). Elevated serum CA125 can also accompany neuroblastoma, and elevated CEA and SCC levels can accompany colorectal cancer. Another marker, the differentiation antigen mesothelin, is expressed on the surfaces of normal mesothelial cells and also on certain cancer cells, including epithelial ovarian tumors and mesotheliomas. Compositions and methods pertaining to mesothelin (Chang et al., 1992 Canc. Res. 52:181; Chang et al., 1992 Int. J. Canc. 50:373; Chang et al., 1992 Int. J. Canc. 51 :548; Chang et al., 1996 Proc. Nat. Acad. Sci. USA 93:136, Chowdhury et al., 1998 Proc. Nat. Acad. Sci. USA 95:669; Yamaguchi et al., 1994 J. Biol. Chem. 269:805; Kojima et al., 1995 J. Biol. Chem. 270:21984) and structurally related mesothulin related antigen (MRA; see, e.g., Scholler et al., 1999 Proc. Nat. Acad. Sci. USA 96:11531) are known in the art, including uses in cancer detection and therapies as described in WO 00/50900 and in U.S. patent 6,770,445. There is a compelling need for additional markers useful in multiple marker diagnostic screening.

The "four-disulfide core" family of proteins comprises a heterogeneous group of small acid- and heat-stable molecules of divergent function and which includes human epididymal four-disulfide core protein, or "HE4" (Kirchhoff et al., 1991 Biol. Reprod. 45:350-357; Wang et al., 1999 Gene 229:101; Schummer et al., 1999 Gene 238:375).

HE4 cDNA was first isolated from human epididymis (Kirchhoff et al., 1991 Biol. Reprod. 45:350-357), and HE4 cDNA was later detected with high frequency in cDNA libraries constructed from ovarian carcinomas (Wang et al., 1999 Gene 229: 101; Schummer et al., 1999 Gene 238:375). HE4a, a new member of the "four-disulfide core" family of proteins was described in U.S. patent application publication number 2003/0108965 A1. HE4a exhibits a sequence that is highly similar to, but distinct from, HE4. HE4a has been described in U.S. patent application publication no. 2003/0108965 A1, which is incorporated herein by reference, including the SEQ ID NO designations used in this disclosure. For the purposes of this disclosure, detection of either HE4 or HE4a are considered synonymous, and detection of either molecule can be used in the methods described herein. It is not material whether HE4a is a molecule distinct from HE4 or whether the sequence of HE4a merely represents a correction of the published HE4 sequence.
WO03021273 describes methods for detection of malignant conditions where HE4a is over expressed, including ovarian cancer. Rosen, et al. (2005) describes that CA 125 as a single maker is not sufficiently sensitive to detect early stage ovarian cancer. US 2005/214826 describes methods to determine whether a patient is afflicted with, responds to therapy, is likely to develop, an ovarian cancer. It also describes a method to differentiate a benign from malignant pelvic mass.

### SUMMARY OF THE DISCLOSURE

In a first aspect, the present invention relates to a method of assessing whether a patient is afflicted with stage I ovarian cancer, the method comprising assessing at least two markers, including both the HE4 marker and another marker selected from the group consisting of SMRP, CA125, and CA72-4, in a biological fluid sample obtained from the patient, wherein elevated levels of the markers are correlated with increased likelihood that the patient is afflicted with stage I ovarian cancer.

In a second aspect, the present invention relates to a method of differentiating whether a pelvic mass in a patient is benign or a stage I ovarian cancer, the method comprising assessing at least two markers, including both the HE4 marker and another marker selected from the group consisting of SMRP, CA125, and CA72-4, in a biological fluid sample obtained from the patient, wherein elevated levels of the markers are correlated with increased likelihood that the pelvic mass is a stage I ovarian cancer.

In a third aspect, the present invention relates to a method of assessing the response of a patient afflicted with stage I ovarian cancer to a treatment, the method comprising assessing at least two markers, including both the HE4 marker and another marker selected from the group consisting of SMRP, CA125, and CA72-4, in biological fluid samples obtained from the patient at different times during treatment, wherein decreased levels of the markers at the later time indicate that the patient is responding to the treatment.

In a fourth aspect, the present invention relates to a method of assessing the likelihood that a patient will develop a stage I ovarian cancer, the method comprising assessing at least two markers, including both the HE4 marker and another marker selected from the group consisting of SMRP, CA125, and CA72-4, in a biological fluid sample obtained from the patient, wherein elevated levels of the markers are correlated with increased likelihood that the patient will develop a stage I ovarian cancer.

In an embodiment of the first aspect, the patient exhibits a pelvic mass.

In an embodiment of the first, second, third and/or fourth aspect, the method comprises assessing the HE4 and CA125 markers. Suitably, when the method is according to the fourth aspect, the patient exhibits normal levels of the CA125 marker and an elevated level of the HE4 marker is correlated with increased likelihood that the patient will develop a stage I ovarian cancer.

In an embodiment of the first, second, third and/or fourth aspect, the method comprises assessing the HE4 and SMRP markers.

In an embodiment of the third aspect, the treatment is intraperitoneal chemotherapy.

In an embodiment of the third aspect, the treatment comprises administration to the patient of an antibody that binds specifically with CA125.
Disclosed herein is a method of assessing whether a patient (e.g., a patient who exhibits a pelvic mass) is afflicted with ovarian cancer. The method comprises assessing at least two markers, including both the HE4 marker and another marker selected from the group consisting of SMRP, CA 125, and CA72-4, in a sample obtained from the patient. Elevated levels of the markers is correlated with increased likelihood that the patient is afflicted with ovarian cancer. By way of example, the HE4 and CA125 markers or the HE4 and SMRP markers can be assessed in a sample. Such methods can be used to differentiate whether a pelvic mass in a patient is benign or an ovarian cancer.

Disclosed herein is a method of assessing the response of a patient afflicted with ovarian cancer to a treatment. The method comprises assessing at least two markers, including both the HE4 marker and another marker selected from the group consisting of SMRP, CA 125, and CA72-4, in samples obtained from the patient at different times during treatment. Decreased levels of the markers at the later time indicates that the patient is responding to the treatment. The the treatment can be intraperitoneal chemotherapy or administration to the patient of an antibody that binds specifically with CA 125, for example.

Disclosed herein is a method of assessing recurrence in a patient who has been treated for ovarian cancer. The method comprises assessing at least two markers, including both the HE4 marker and another marker selected from the group consisting of SMRP, CA 125, and CA72-4, in a sample obtained from the patient following treatment. Elevated levels of the markers indicates that the ovarian cancer is recurring in the patient. The markers can be assessed multiple times following the treatment, and increasing levels of the markers indicates that the ovarian cancer is recurring in the patient.

Disclosed herein is a method of assessing the likelihood that a patient will develop an ovarian cancer. The method comprises assessing at least two markers, including both the HE4 marker and another marker selected from the group consisting of SMRP, CA125, and CA72-4, in a sample obtained from the patient. Elevated levels of the markers is correlated with increased likelihood that the patient will develop an ovarian cancer.

Disclosed herein are methods of staging and/or grading a tumor in a patient who is afflicted with ovarian cancer. The method comprising assessing at least two markers, including both the HE4 marker and another marker selected from the group consisting of SMRP, CA 125, and CA72-4, in a sample obtained from the patient. Increasing levels of the markers are correlated with more advanced stages of ovarian cancer and/or with more higher grades of ovarian cancer.

### BRIEF SUMMARY OF THE SEVERAL TABLES

Table 1 includes the results of example 1 summarized by a comparison of sensitivities of markers used at set specificity levels.
Table 2 includes the age distribution in subjects with and without malignancy, as described in Example 2.
Table 3 includes the clinical diagnosis of subjects with benign disease, as described in Example 2.
Table 4 includes the histology and stage distribution of subjects with ovarian cancers, as described in Example 2.
Table 5 includes the mean and medium marker values, comparing ovarian and benign tumors, as described in Example 2.
Table 6 includes a comparison of sensitivity values of marker combinations at set specificity levels, as described in Example 2.
Table 7 includes a comparison of tumor markers for subject with stage I ovarian cancer or benign disease, as described in Example 2.
Table 8 includes a comparison of sensitivities of tumor marker combination at set specificity levels of 90%, 95% and 98% for subjects with benign and stage I ovarian cancer as described in Example 2.
Table 9 summarizes the sensitivity values for single marker and marker combinations at set specificity levels of 90%, 95%, and 98% in subjects with benign disease as described in Example 3.

### DETAILED DESCRIPTION

The subject matter of this disclosure relates generally to assessment of ovarian cancer in women using a combination of biological markers. Assessing HE4 alone or in combination with one or more additional markers of ovarian cancer (especially markers CA 125, SMRP, and CA72-4) can be used to diagnose occurrence of an ovarian cancer in a human patient, and to assess the response of that cancer to treatments of various types (e.g., intraperitoneal chemotherapy or treatment with an anti-CA125 antibody such as the OVAREX® product of ViRexx company of Edmonton, Alberta, Canada). Furthermore, assessment of these markers can be used to monitor recurrence of an ovarian cancer in a patient or to assess the likelihood that a patient will develop an ovarian cancer.

### Definitions

As used herein by the term a "sample" is meant material which can be specifically related to a patient and from which specific information about the patient can be determined, calculated or inferred. A sample can be composed in whole or in part of biological material from of the patient. A sample can also be material that has contacted the patient in a way that allows tests to be conducted on the sample which provides information about the patient. A sample may also be material that has contacted other material that is not of the patient but allows the first material to then be tested to determine information about the patient. A sample can contact sources of biologic material other than the patient provided that one skilled in the art can nevertheless determine information about the patient from the sample. It is also understood that extraneous material or information that is not the sample could be utilized to conclusively link the patient to the sample. For a non-limiting example, a double blind test requires a chart or database to match a sample with a patient.

As used herein by the term "patient" it is meant a biologic organism that is the subject of an assay to determine information about the organism. While, in most examples of the methods described herein the patient is a human, those methods are not limited for use with an individual human. The patient can be a group of humans. The patient can also be part of a human. For non-limiting examples, the patient could be a tissue sample not linked to a human body, or a transformed cell line. In certain examples the patient might also a non-human organism.

As used herein the term "reference value" it is meant a value that statistically correlates to a particular outcome when compared to an assay result. In preferred examples the reference value is determined from statistical review of studies that compare HE4a expression with known clinical outcomes. Some such studies are presented in the Examples section herein. However, studies from the literature and the experience of users of the methods described herein experience can also be used to produce or adjust a reference value. Reference value can also be determined from consideration of cases and results that are particularly relevant to the patient's medical history, genetics, age and other factors.

As used herein the term "body fluid" it is meant a material obtained from a patient that is substantially fluid in consistency, but may have solid or particulate matter associated with it. A body fluid can also contain material and portions that are not from the patient. For instance a body fluid can be diluted with water, or can contain preservative, such as EDTA. Non-limiting examples of body fluids blood, serum, serosal fluids, plasma, lymph, urine, cerebrospinal fluid, saliva, mucosal secretions of the secretory tissues and organs, vaginal secretions, breast milk, tears, and ascites fluids such as those associated with non-solid tumors. Additional examples include fluids of the pleural, pericardial, peritoneal, abdominal and other body cavities, and the like. Biological fluids may further include liquid solutions contacted with a subject or biological source, for example, cell and organ culture medium including cell or organ conditioned medium, lavage fluids and the like.

A sample is obtained from a patient "during treatment" if the sample is obtained as a prelude to administration, at the time of administration, or following administration of a therapeutic composition or method or during the period of follow-up that occurs thereafter. Use of this term explicitly encompassess situations in which samples are obtained prior to and after administration of the treatment (i.e., to assess effectiveness of the treatment or recurrence), as well as situations in which multiple samples are taken intermittently during an extended course of treatment.

### Detailed description

Ovarian cancer is the most lethal of the gynecologic malignancies. It is estimated over 22,000 women will be diagnosed and over 16,000 will die of disease in 2005 (Murray et al., 2005. Ca. Cancer. J. Clin. 2005; 55(1): 10-30). The majority of these women present with an adnexal mass with or without evidence of disease in the upper abdomen. However, twenty percent of all women in the US will be diagnosed with an adnexal mass or cyst at some point, with only a small percentage of these representing a malignancy.

Ovarian cancer is surgically staged, and studies show that patients operated on by gyn-oncologists are more often adequately staged and debulked than those operated on by non-gyn-oncologists. Clinical decisions must be frequently made as to when a patient with an adnexal mass should be referred to a gyn-oncologist for surgical exploration, as opposed to undergoing surgery by a benign gynecologist. The CA 125 tumor marker can be used to help predict which patients with a pelvic mass may have ovarian cancer. Unfortunately, many benign gynecologic and non-gynecologic conditions also elevate CA125, decreasing its specificity; a normal CA125 is also found in up to half of patients with early stage ovarian cancer. A more accurate test is needed to determine the risk of malignancy in patients presenting with an adnexal mass and thus help appropriately triage patients to a gyn-oncologist.

The poor survival rate in ovarian cancer is due in part to the lack of effective screening coupled with the lack of symptoms in early stage disease. As a result, 70% of patients have Stage 111 or IV disease at the time of diagnosis with a 5 year survival rate of approximately 44% (Murry et al ibid). For early stage disease (stage 1) the five year survival rate ranges from 74 to 90% (Averette et al., 1995, Cancer 76(6): 1096-1103; Young et al, 1990 N. Engl. J. Med., 322(15): 1021-1027). Unfortunately, an effective screening test for ovarian cancer does not currently exist and tumor markers with an increased sensitivity and specificity are needed. A marker, or marker combination, that can adequately distinguish benign from malignant masses would also be a good candidate for evaluation as a potential screening test.

The serum tumor marker CA125 has been the most widely used marker in ovarian cancer, however, it is neither sufficiently sensitive nor specific for the detection of early stage disease (Einhorn et al., 1992, Hematol. Oncol. Clin. North. Am. 6(4):843- 850; Campbell et al., 1990, Br. J. Obstet. Gynaecol. 97(4): 304- 311; Jacobs et al., 1993, BMJ 306(6884): 1030-1034; DePriest et al., 1993, Gynecol. Oncol 51 (2): 205- 209). Disappointingly, the combined use of ultrasound imaging and CA125 serum levels in the assessment of high risk patients has not been shown to decrease the mortality from this disease (Jacobs et al., 1990, Br. J. Obstet. Gynaecol. 97(10): 922- 929; Karlan et al, 1993 Am. J. Obstet. Gynecol. 169(3): 494- 501 ; Bourne et al, 1993, BMJ 306(6884): 1025- 1029; Schwartz et al., 1991, Yale J. Biol. Med. 64(6):557- 571). Several novel tumor markers have also be evaluated to determine their utility in identifying ovarian carcinomas.

The levels of soluble mesothelin related peptides (SMRP) and HE4 have recently been found to be elevated in women with ovarian cancer(Schaner et al., 2003, Mol. Biol. Cell 14(11): 4376- 4386; Hough et al., 2000, Cancer Res. 60(22): 6281 - 6287; Scholler et al., 1999, Proc. Natl. Acad. Sci. U. S. A. 96(20): 1 1531 - 1 1536; Hellstrom et al., 2003, Cancer Res. 63(13): 3695- 3700). Soluble mesothelin-related peptides are members of the megakaryocyte potentiating factor family and have been detected in both the serum and urine of patients with ovarian cancer14. In a preliminary study, serum SMRP levels were elevated in 92% of women with ovarian cancer as compared to 10% of those with benign gynecological conditions. Using a urine assay, SMRP levels were elevated in 67% of those with ovarian cancer compared to 14% of healthy volunteers and 20% of those with benign gynecologic conditions (Bones et al., 2003, American Association for Clinical Chemisty, 55th Annual Meeting).

Osteopontin has recently been identified by microarray techniques as a potential ovarian cancer marker, and has been found to be elevated in the serum of ovarian cancer patients (Kim et al,, ibid). Kim et al, has shown both increased tissue expression of osteopontin as well as increased plasma levels in patients with ovarian cancer; however to obtain a sensitivity of 80% specificity fell to only 80% (Kim et al., ibid). CA72-4 is a protein initially described in the early 1980's that has been shown to be elevated in a variety of carcinomas (Colcher et al., Proc. Nat. Acad. Sci. 78(5): 3199- 3203), including ovarian, when used as a single marker and when combined with CA125 (Negishi et al., 1993, Gynecol Oncol. 48: 148-154; Skates et al., 2004, J. Clinical Oncology 22(20): 4059- 4066; Fayed et al., 1998, Disease Markers 14: 155- 160). Activin and inhibin have also been investigated as possible markers, although sensitivity has not been high in epithelial cancer (Robertson et al., 2002, Molecular and Cellular Endocrinology 191(1): 97- 103; Robertson et al., 2004, Endocrine-Related Cancer, 11: 35-49).

HE4 is an 11kDa protein that is a precursor to the epididymal secretory protein E4 with the gene initially identified using microarray technology. In normal ovarian tissue there is only minimal gene expression with elevated expression, in ovarian carcinomas. Serum HE4 levels were elevated in 88% of women with ovarian cancer compared to 5% of those with benign gynecologic conditions (Kim et al., 2003, JAMA 287(13)1671- 1679). Interestingly, the elevations in both SMRP and HE4 assay levels were independent of disease stage and menopausal status. Using the urinary assay, SMRP levels were elevated in 50% of Stage I patients and 79% of Stage II patients, raising the possibility of increased detection of early stage disease (Bones et al., ibid).

The methods described herein pertain to HE4a, a member of the "four-disulfide core" family of proteins as described herein, which exhibits a sequence that is highly similar to, but distinct from, HE4 (Kirchhoff et al., 1991 Biol. Reproduct. 45:350-357). As described herein, HE4a (and not HE4) is unexpectedly shown to be overexpressed in certain malignancies, for example in endometrial carcinomas, as well as in a number of other human tissues, in marked contrast to the restricted expression pattern of HE4 in human epididymal epithelial cells (Kirchhoff et al., 1991), The methods described herein also pertains in part to compositions and methods for detection of cell surface and/or soluble forms of HE4a that occur naturally in subjects, including elevated levels of such polypeptides in subjects having certain carcinomas (e.g., endometrial carcinomas). This disclosure therefore provides useful compositions and methods for the detection and diagnosis of a malignant condition in a subject by specific detection of such cell surface and/or soluble HE4a polypeptides.

According to the methods described herein, a soluble human HE4a antigen polypeptide (or HE4a polypeptide) can be detected in a biological sample from a subject or biological source. Biological samples may be provided by obtaining a blood sample, biopsy specimen, tissue explant, organ culture, biological fluid or any other tissue or cell preparation from a subject or a biological source. The subject or biological source may be a human or non-human animal, a primary cell culture or culture adapted cell line including but not limited to genetically engineered cell lines that may contain chromosomally integrated or episomal recombinant nucleic acid sequences, immortalized or immortalizable cell lines, somatic cell hybrid cell lines, differentiated or differentiable cell lines, transformed cell lines and the like. In certain preferred examples of the methods described herein, the subject or biological source may be suspected of having or being at risk for having a malignant condition, which in certain further preferred examples may be an endometrial cancer such as an endometrial carcinoma and in certain other preferred examples of the methods described herein the subject or biological source may be known to be free of a risk or presence of such diseases.

In some examples, the biological sample includes at least one cell from a subject or biological source, and in certain other preferred examples the biological sample is a biological fluid containing another tumor marker, such as CA-125 or a soluble human mesothelin related antigen polypeptide. Biological fluids are typically liquids at physiological temperatures and may include naturally occurring fluids present in, withdrawn from, expressed or otherwise extracted from a subject or biological source. Certain biological fluids derive from particular tissues, organs or localized regions and certain other biological fluids may be more globally or systemically situated in a subject or biological source. Non-limiting examples of biological fluids include blood, serum and serosal fluids, plasma, lymph, urine, cerebrospinal fluid, saliva, mucosal secretions of the secretory tissues and organs, vaginal secretions, breast milk, tears, and ascites fluids such as those associated with non-solid tumors. Additional examples include fluids of the pleural, pericardial, peritoneal, abdominal and other body cavities, and the like. Biological fluids may further include liquid solutions contacted with a subject or biological source, for example, cell and organ culture medium including cell or organ conditioned medium, lavage fluids and the like. In other preferred examples the biological sample is a cell-free liquid solution, such as blood serum, plasma, or the supernatant of centrifuged urine.

In certain other preferred examples the biological sample comprises an intact cell, and in certain other preferred examples the biological sample comprises a cell extract containing a nucleic acid sequence encoding a HE4a antigen polypeptide having the amino acid sequence set forth in SEQ ID NOS: 11 or 13, or a fragment or variant thereof. In still other examples of the methods described herein, it is desired that cells are physically or chemically ruptured or lysed before assaying to provide cell contents for analysis.

A "molecule naturally occurring in soluble form" in a sample may be a soluble protein, polypeptide, peptide, amino acid, or derivative thereof; a lipid, fatty acid or the like, or derivative thereof; a carbohydrate, saccharide or the like or derivative thereof, a nucleic acid, nucleotide, nucleoside, purine, pyrimidine or related molecule, or derivative thereof, or the like; or any combination thereof such as, for example, a glycoprotein, a glycolipid, a lipoprotein, a proteolipid, or any other biological molecule that is a soluble or cell-free constituent of a biological sample as provided herein. A "molecule naturally occurring in soluble form" further refers to a molecule that is in solution or present in a biological sample, including a biological fluid as provided herein, and that is not bound to the surface of an intact cell. For example, a molecule naturally occurring in soluble form may include but need not be limited to a solute; a component of a macromolecular complex; a material that is shed, secreted or exported from a cell; a colloid; a microparticle or nanoparticle or other fine suspension particle, or the like.

The presence of a malignant condition in a subject refers to the presence of dysplastic, cancerous and/or transformed cells in the subject, including, for example neoplastic, tumor, non-contact inhibited or oncogenically transformed cells, or the like. By way of illustration and not limitation, in the context of the present methods described herein a malignant condition may refer further to the presence in a subject of cancer cells that are capable of secreting, shedding, exporting or releasing a HE4a antigen polypeptide (or a HE4a polypeptide) in such a manner that elevated levels of such a polypeptide are detectable in a biological sample from the subject. In preferred examples, for example, such cancer cells are malignant epithelial cells such as carcinoma cells, and in particularly preferred examples such cancer cells are malignant mesothelioma cells, which are transformed variants of squamous cell epithelial or mesothelial cells that are found, for example, lining pleural, pericardial, peritoneal, abdominal and other body cavities.

In the most preferred examples of the methods described herein, tumor cells, the presence of which signifies the presence of a malignant condition, are endometrial carcinoma cells, including primary and metastatic endometrial carcinoma cells. Criteria for classifying a malignancy as endometrial carcinoma are well known in the art as are the establishment and characterization of human endometrial carcinoma cell lines from primary and metastatic tumors. In other examples, the malignant condition may be mesothelioma, pancreatic carcinoma, non-small cell lung carcinoma or another form of cancer, including any of the various carcinomas such as squamous cell carcinomas and adenocarcinomas, and also including sarcomas and hematologic malignancies (e.g., leukemias, lymphomas, myelomas, etc.). Classification of these and other malignant conditions is known to those having familiarity with the art, and the present disclosure provides determination of the presence of a HE4a polypeptide in such a malignant condition without undue experimentation.

Reference values are provided in the examples contained herein. Such values are suitable for practice of the methods described herein. However it should be noted that the use of the methods described herein is not limited to those reference values or that data. Those skilled in the art can obtain a reference value for their particular needs. Such a reference value can be obtained by analyzing HE4a express of patients as they undergo biopsy procedures for uterine or endometrial masses suspected of being malignant. Methods of obtaining such reference values are contained herein and provided in the examples. Users of the methods described herein may will to obtain different reference value than provided herein to focus on specific categories of patients. In is foreseen that such categories could include age, genetic background, risk of cancer, medical history, blood type, physical characteristics such as body mass, and other categories.

As provided herein, the method of screening for the presence of a malignant condition in a subject may feature the use of an antibody specific for a HE4a antigen polypeptide or an antibody specific for a HE4a polypeptide.

Antibodies that are specific for a HE4a antigen polypeptide (or a HE4a polypeptide) are readily generated as monoclonal antibodies or as polyclonal antisera, or may be produced as genetically engineered immunoglobulins (Ig) that are designed to have desirable properties using methods well known in the art For example, by way of illustration and not limitation, antibodies may include recombinant IgGs, chimeric fusion proteins having immunoglobulin derived sequences or "humanized" antibodies (see. e.g.. U.S. Pat. Nos. 5,693,762; 5,585,089, 4,816,567; 5,225,539; 5,530,101; and references cited therein) that may all be used for detection of a human HE4a polypeptide according to the methods described herein. Such antibodies may be prepared as provided herein, including by immunization with HE4a polypeptides as described below. For example, as provided herein, nucleic acid sequences encoding HE4a polypeptides are disclosed, such that those skilled in the art may routinely prepare these polypeptides for use as immunogens. For instance, monoclonal antibodies such as 2H5, 3D8 and 4H4, which are described in greater detail below, may be used to practice certain methods according to the methods described herein.

The term "antibodies" includes polyclonal antibodies, monoclonal antibodies, fragments thereof such as F(ab¹)₂, and Fab fragments, as well as any naturally occurring or recombinantly produced binding partners, which are molecules that specifically bind a HE4a polypeptide. Antibodies are defined to be "immunospecific" or specifically binding if they bind HE4a polypeptide with a Kₐ of greater than or equal to about 10.sup.4 M.sup.⁻¹, preferably of greater than or equal to about 10⁵ M⁻¹, more preferably of greater than or equal to about 10⁶ M⁻¹ and still more preferably of greater than or equal to about 10.sup.⁷ M⁻¹. Affinities of binding partners or antibodies can be readily determined using conventional techniques, for example those described by Scatchard et al., Ann. N.Y. Acad. Sci. 51:660 (1949). Determination of other proteins as binding partners of a HE4a polypeptide can be performed using any of a number of known methods for identifying and obtaining proteins that specifically interact with other proteins or polypeptides, for example, a yeast two-hybrid screening system such as that described in U.S. Pat. No. 5,283, 173 and U.S. Pat. No 5,468,614, or the equivalent. The methods described herein also includes the use of a HE4a polypeptide, and peptides based on the amino acid sequence of a HE4a polypeptide, to prepare binding partners and antibodies that specifically bind to a HE4a polypeptide.

Antibodies may generally be prepared by any of a variety of techniques known to those of ordinary skill in the art (see, e.g., Harlow and Lane, Antibodies: A Laboratory Manual, Cold Spring Harbor Laboratory, 1988). In one such technique, an immunogen comprising a HE4a polypeptide, for example a cell having a HE4a polypeptide on its surface or an isolated HE4a polypeptide is initially injected into a suitable animal (e g., mice, rats, rabbits, sheep and goats), preferably according to a predetermined schedule incorporating one or more booster immunizations, and the animals are bled periodically. Polyclonal antibodies specific for the HE4a polypeptide may then be purified from such antisera by, for example, affinity chromatography using the polypeptide coupled to a suitable solid support.

Monoclonal antibodies specific for HE4a polypeptides or variants thereof may be prepared, for example, using the technique of Kohler and Milstein (1976 Eur. J. Immunol. 6:511-519), and improvements thereto. Briefly, these methods involve the preparation of immortal cell lines capable of producing antibodies having the desired specificity (i.e., reactivity with the mesothelin polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. For example, the spleen cells and myeloma cells may be combined with a membrane fusion promoting agent such as polyethylene glycol or a nonionic detergent for a few minutes, and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred. Hybridomas that generate monoclonal antibodies that specifically bind to HE4a polypeptides are contemplated by the methods described herein.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse or other suitable host. Monoclonal antibodies may then be harvested from the ascites fluid or the blood. Contaminants may be removed from the antibodies by conventional techniques, such as chromatography, gel filtration, precipitation, and extraction. For example, antibodies may be purified by chromatography on immobilized Protein G or Protein A using standard techniques.

Within certain examples, the use of antigen-binding fragments of antibodies may be preferred. Such fragments include Fab fragments, which may be prepared using standard techniques (e.g., by digestion with papain to yield Fab and Fc fragments). The Fab and Fc fragments may be separated by affinity chromatography (e.g., on immobilized protein A columns), using standard techniques. Such techniques are well known in the art, see, e.g., Weir, D. M., Handbook of Experimental Immunology, 1986, Blackwell Scientific, Boston.

Multifunctional fusion proteins having specific binding affinities for pre-selected antigens by virtue of immunoglobulin V-region domains encoded by DNA sequences linked in-frame to sequences encoding various effector proteins are known in the art, for example, as disclosed in EP-B1-0318554, U.S. Pat. No. 5, 132,405, U.S. Pat. No. 5,091,513 and U.S. Pat. No. 5,476,786. Such effector proteins include polypeptide domains that may be used to detect binding of the fusion protein by any of a variety of techniques with which those skilled in the art will be familiar, including but not limited to a biotin mimetic sequence (see, e.g., Luo et al., 1998 J. Biotechnol. 65:225 and references cited therein), direct covalent modification with a detectable labeling moiety, non-covalent binding to a specific labeled reporter molecule, enzymatic modification of a detectable substrate or immobilization (covalent or non-covalent) on a solid-phase support.

Single chain antibodies for use in the methods described herein may also be generated and selected by a method such as phage display (see, e.g., U.S. Pat. No. 5,223,409; Schlebusch et al., 1997 Hybridoma 16:47; and references cited therein). Briefly, in this method, DNA sequences are inserted into the gene III or gene VIII gene of a filamentous phage, such as M13. Several vectors with multicloning sites have been developed for insertion (McLafferty et al., Gene 128:29-36, 1993; Scott and Smith, Science 249:386-390, 1990; Smith and Scott Methods Enzymol. 217:228-257, 1993). The inserted DNA sequences may be randomly generated or may be variants of a known binding domain for binding to a HE4a polypeptide. Single chain antibodies may readily be generated using this method. Generally, the inserts encode from 6 to 20 amino acids. The peptide encoded by the inserted sequence is displayed on the surface of the bacteriophage. Bacteriophage expressing a binding domain for a HE4a polypeptide are selected by binding to an immobilized HE4a polypeptide, for example a recombinant polypeptide prepared using methods well known in the art and nucleic acid coding sequences as disclosed herein. Unbound phage are removed by a wash, typically containing 10 mM Tris, 1 mM EDTA, and without salt or with a low salt concentration. Bound phage are eluted with a salt containing buffer, for example. The NaCl concentration is increased in a step-wise fashion until all the phage are eluted. Typically, phage binding with higher affinity will be released by higher salt concentrations. Eluted phage are propagated in the bacteria host. Further rounds of selection may be performed to select for a few phage binding with high affinity. The DNA sequence of the insert in the binding phage is then determined. Once the predicted amino acid sequence of the binding peptide is known, sufficient peptide for use herein as an antibody specific for a HE4a polypeptide may be made either by recombinant means or synthetically. Recombinant means are used when the antibody is produced as a fusion protein. The peptide may also be generated as a tandem array of two or more similar or dissimilar peptides, in order to maximize affinity or binding.

To detect an antigenic determinant reactive with an antibody specific for a HE4a polypeptide, the detection reagent is typically an antibody, which may be prepared as described herein. There are a variety of assay formats known to those of ordinary skill in the art for using an antibody to detect a polypeptide in a sample, including but not limited to enzyme linked immunosorbent assay (ELISA), radioimmunoassay (RLA.), immunofluorimetry, immunoprecipitation, equilibrium dialysis, immunodiffusion and other techniques. See, e.g., Harlow and Lane, Antibodies: A Laboratory Manual, Cold Spring Harbor Laboratory, 1988; Weir, D. M., Handbook of Experimental Immunology, 1986, Blackwell Scientific, Boston. For example, the assay may be performed in a Western blot format, wherein a protein preparation from the biological sample is submitted to gel electrophoresis, transferred to a suitable membrane and allowed to react with the antibody. The presence of the antibody on the membrane may then be detected using a suitable detection reagent, as is well known in the art and described below.

In another example, the assay involves the use of an antibody immobilized on a solid support to bind to the target HE4a polypeptide and remove it from the remainder of the sample. The bound HE4a polypeptide may then be detected using a second antibody reactive with a distinct HE4a polypeptide antigenic determinant, for example, a reagent that contains a detectable reporter moiety. As a non-limiting example, according to this example the immobilized antibody and the second antibody which recognize distinct antigenic determinants may be any two of the monoclonal antibodies described herein selected from the monoclonal antibodies 2H5, 3D8 and 4H4. Alternatively, a competitive assay may be utilized, in which a HE4a polypeptide is labeled with a detectable reporter moiety and allowed to bind to the immobilized HE4a polypeptide specific antibody after incubation of the immobilized antibody with the sample. The extent to which components of the sample inhibit the binding of the labeled polypeptide to the antibody is indicative of the reactivity of the sample with the immobilized antibody, and as a result, indicative of the level of HE4a in the sample.

The solid support may be any material known Io those of ordinary skill in the art to which the antibody may be attached, such as a test well in a microtiter plate, a nitrocellulose filter or another suitable membrane. Alternatively, the support may be a bead or disc, such as glass, fiberglass, latex or a plastic such as polystyrene or polyvinylchloride. The antibody may be immobilized on the solid support using a variety of techniques known to those in the art, which are amply described in the patent and scientific literature.

In certain preferred examples, the assay for detection of HE4a antigen polypeptide in a sample is a two-antibody sandwich assay. This assay may be performed by first contacting a HE4a polypeptide-specific antibody (e.g., a monoclonal antibody such as 2H5, 3D8 or 4H4) that has been immobilized on a solid support, commonly the well of a microtiter plate, with the biological sample, such that a soluble molecule naturally occurring in the sample and having an antigenic determinant that is reactive with the antibody is allowed to bind to the immobilized antibody (e.g., a 30 minute incubation time at room temperature is generally sufficient) to form an antigen-antibody complex or an immune complex. Unbound constituents of the sample are then removed from the immobilized immune complexes. Next, a second antibody specific for a HE4a antigen polypeptide is added, wherein the antigen combining site of the second antibody does not competitively inhibit binding of the antigen combining site of the immobilized first antibody to a HE4a polypeptide (e.g., a monoclonal antibody such as 2H5, 3D8 or 4H4 that is not the same as the monoclonal antibody immobilized on the solid support). The second antibody may be detectably labeled as provided herein, such that it may be directly detected. Alternatively, the second antibody may be indirectly detected through the use of a detectably labeled secondary (or "second stage") anti-antibody, or by using a specific detection reagent as provided herein. The methods described herein are not limited to any particular detection procedure, as those having familiarity with immunoassays will appreciate that there are numerous reagents and configurations for immunologically detecting a particular antigen (e.g., a mesothelin polypeptide) in a two-antibody sandwich immunoassay.

In certain preferred examples of the methods described herein using the two-antibody sandwich assay described above, the first, immobilized antibody specific for a HE4a antigen polypeptide is a polyclonal antibody and the second antibody specific for a HE4a antigen polypeptide is a polyclonal antibody. Any combination of non-competitive HE4a antibodies could be used with the methods described herein. Including monoclonal antibodies, polyclonal antibodies and combinations thereof. In certain other examples of the methods described herein the first, immobilized antibody specific for a HE4a antigen polypeptide is a monoclonal antibody and the second antibody specific for a HE4a antigen polypeptide is a polyclonal antibody. In certain other examples of the methods described herein the first, immobilized antibody specific for a HE4a antigen polypeptide is a polyclonal antibody and the second antibody specific for a HE4a antigen polypeptide is a monoclonal antibody. In certain other highly preferred examples of the methods described herein the first, immobilized antibody specific for a HE4a antigen polypeptide is a monoclonal antibody and the second antibody specific for a HE4a antigen polypeptide is a monoclonal antibody. For example, in these examples it should be noted that monoclonal antibodies 2H5, 3D8 and 4H4 as provided herein recognize distinct and noncompetitive antigenic determinants (e.g., epitopes) on HE4a polypeptides., such that any pairwise combination of these monoclonal antibodies may be employed. In other preferred examples of the methods described herein the first, immobilized antibody specific for a HE4a antigen polypeptide and/or the second antibody specific for a HE4a antigen polypeptide may be any of the kinds of antibodies known in the art and referred to herein, for example by way of illustration and not limitation, Fab fragments. F(ab¹).sub.2 fragments, immunoglobulin V-region fusion proteins or single chain antibodies. Those familiar with the art will appreciate that the methods described herein encompass the use of other antibody forms, fragments, derivatives and the like in the methods disclosed and claimed herein.

In certain particularly preferred examples, the second antibody may contain a detectable reporter moiety or label such as an enzyme, dye, radionuclide, luminescent group, fluorescent group or biotin, or the like. Any reporter moiety or label could be used with the methods described herein, so long as the signal of such is directly related or proportional to the quantity of antibody remaining on the support after wash. The amount of the second antibody that remains bound to the solid support is then determined using a method appropriate for the specific detectable reporter moiety or label. For radioactive groups, scintillation counting or autoradiographic methods are generally appropriate. Antibody-enzyme conjugates may be prepared using a variety of coupling techniques (for review see, e.g., Scouten, W. H., Methods in Enzymology 135:30-65, 1987). Spectroscopic methods may be used to detect dyes (including, for example, colorimetric products of enzyme reactions), luminescent groups and fluorescent groups, Biotin may be detected using avidin or streptavidin, coupled to a different reporter group (commonly a radioactive or fluorescent group or an enzyme). Enzyme reporter groups may generally be detected by the addition of substrate (generally for a specific period of time), followed by spectroscopic, spectrophotometric or other analysis of the reaction products. Standards and standard additions may be used to determine the level of antigen in a sample, using well known techniques.

In another examples, the methods described herein involve use of a HE4a antigen polypeptide as provided herein to screen for the presence of a malignant condition by detection of immunospecifically reactive antibodies in a biological sample from a biological source or subject. According to this example, a HE4a antigen polypeptide (or a fragment or variant thereof including a truncated HE4a antigen polypeptide as provided herein) is detectably labeled and contacted with a biological sample to detect binding to the HE4a antigen polypeptide of an antibody naturally occurring in soluble form in the sample. For example, the HE4a antigen polypeptide may be labeled biosynthetically by using the sequences disclosed herein in concert with well known methods such as incorporation during in vitro translation of a readily detectable (e.g. radioactively labeled) amino acid, or by using other detectable reporter moieties such as those described above. Without wishing to be bound by theory, this example of the methods described herein contemplates that certain HE4a polypeptides such as the HE4a fusion polypeptides disclosed herein, may provide peptides that are particularly immunogenic and so give rise to specific and detectable antibodies. For example, according to this theory certain HE4a fusion polypeptides may represent "non-self" antigens that provoke an avid immune response, while HE4a polypeptides that lack fusion domains may be viewed by the immune system as more resembling "self" antigens that do not readily elicit humoral or cell-mediated immunity.

As noted above, the present methods described herein pertain in part to the surprising finding that soluble forms of HE4a antigen polypeptides occur naturally in subjects, including elevated levels of such soluble HE4a polypeptides in subjects having certain carcinomas.

A method of screening for the presence of a malignant condition according to the methods described herein may be further enhanced by the detection of more than one tumor associated marker in a biological sample from a subject. Accordingly, in certain examples the methods described herein provide a method of screening that, in addition to detecting reactivity of a naturally occurring soluble sample component with an antibody specific for a HE4a antigen polypeptide, also includes detection of at least one additional soluble marker of a malignant condition using established methods as known in the art and provided herein. As noted above, there are currently a number of soluble tumor associated antigens that are detectable in samples of readily obtained biological fluids, for example, certain examples of the methods described herein relate to human mesothelin polypeptides, which include polypeptides such as the novel soluble mesothelin related antigen (MRA) polypeptide described in Scholler et al. (1999 Proc. Nat. Acad. Sci. USA 96: 11531) and as also described in U.S. Patent 6,770,445.

As provided herein, a "mesothelin polypeptide" is a soluble polypeptide having an amino acid sequence that includes the polypeptide sequence EVEKTACPSGKKAREIDES (SEQ ID NO: 14) and further having at least one antigenic determinant reactive with at least one antibody having an antigen combining site that competitively inhibits the immunospecific binding of MAb K-1 (Chang et al, 1996 Proc. Nat. Acad. Sci. USA 93: 136; MAb K-1 is available from, e.g., Signet Laboratories, Inc., Dedham, Mass.) or of monoclonal antibodies OV569, 4H3, 3G3 or 1A6 as provided in U.S. Patent 6,770,445.

Thus, these additional soluble tumor associated antigens for use according to the methods described herein may include, but need not be limited to, mesothelin and mesothelin related antigen, CEA, CA125, sialyl TN, SCC, TPS and PLAP, (see e.g., Bast et al., 1983, N. Eng. J. Med. 309:883; Lloyd et al., 1997, Int. J. Canc. 71:842; Sarandakou et al., 1997, Acta Oncol. 36:755; Sarandakou et al., 1998, Eur. J. Gynaecol. Oncol. 19:73; Meier et al., 1997, Anticanc, Res. 17(4B):2945; Kudoh et al., 1999, Gynecol. Obstet. Invest. 47:52; Ind et al, 1997, Br. J. Obstet. Gynaecol, 104:1024; Bell et al. 1998, Br. J. Obstet. Gynaecol. 105: 1136; Cioffi et al., 1997, Tumori 83:594; Meier et al. 1997, Anticanc. Res. 17(4B):2949; Meier et al., 1997, Anticanc Res. 17(4B):3019) and may further include any known marker the presence of which in a biological sample may be correlated with the presence of at least one malignant condition, as provided herein.

Alternatively, nucleic acid sequences encoding HE4a polypeptides may be detected, using standard hybridization and/or polymerase chain reaction (PCR) techniques. Suitable probes and primers may be designed by those of ordinary skill in the art based on the HE4a cDNA sequences provided herein. Assays may generally be performed using any of a variety of samples obtained from a biological source, such as eukaryotic cells, bacteria, viruses, extracts prepared from such organisms and fluids found within living organisms.

### EXAMPLES

### General methods

Standard recombinant DNA and molecular cloning techniques used in the Examples are well known in the art and are described by Sambrook, J., Fritsch, E. F. and Maniatis, T. Molecular Cloning: A Laboratory Manual; Cold Spring Harbor Laboratoiy Press: Cold Spring Harbor, (1989) (Maniatis) and by T. J. Silhavy, M. L. Bennan, and L. W. Enquist, Experiments with Gene Fusions, Cold Spring Harbor Laboratory, Cold Spring Harbor, N.Y. (1984) and by Ausubel, F. M. et al., Current Protocols in Molecular Biology, pub. by Greene Publishing Assoc, and Wiley- Interscience (1987).

The meaning of abbreviations is as follows: "h" means hour(s), "min" means minute(s), "sec" means second(s), "d" means day(s), "mL" means milliliters, "L" means liters, "U" means units, "pM" means picomolar.

The subject matter of this disclosure is now described with reference to the following Examples. These Examples are provided for the purpose of illustration only.

### Example 1

Development of a multiple marker assay using novel serum tumor markers for the detection of ovarian carcinoma.

Twenty percent of all women in the US will be diagnosed with an adnexal mass or cyst, with a small percentage of these representing a malignancy. Ovarian cancer is surgically staged and studies show that patients operated on by gyn oncologists are more often adequately staged and debulked than those operated on by non-gyn oncologists. The CA125 tumor marker can help predict which patients with a pelvic mass may have ovarian cancer. However, many benign gynecologic conditions also elevate CA125, decreasing its specificity. A more accurate test is needed to help triage these patients to centers with expertise in treating this disease. This approach applies several novel tumor markers to develop a multiple marker assay to predict the risk of ovarian cancer and help triage patients with a pelvic mass to a gyn oncologists.

The methods used in the experiments of this example are now described.

This was an IRB approved prospective trial. After obtaining informed consent serum samples were obtained preoperatively from women undergoing surgery for an adnexal mass and analyzed for levels of CA125, SMRP, HE4, CA72-4, and osteopontin. All pathology results were compared to the tumor markers. Logistic regression models were estimated for each marker individually, and all combinations of these markers. Cross-validation analysis was performed to obtain the sensitivity at both a 95% and 98% specificity.

The results obtained from the experiments of this example are now described.

179 patients were analyzed; 50 ovarian cancers (14 Stage I) and 129 benign ovarian masses. Values for HE4, SMRP, CA125, and CA72-4 all differed between benign masses and cancer. Combining all markers, HE4, SMRP, and CA 125 remained predictive with an AUC of 92%. When all potential marker combinations were compared at a 95% specificity, the combination of CA125 and HE4 had the highest sensitivity at 74.5%. The addition of HE4 to CA125 increased the sensitivity of the test by 7.4%.

These data are summarized in Table 1 .

**Table 1**

| Panels | Cross-validated Sensitivity | |
|---|---|---|
| | 95% Specificity | 98% Specificity |
| *Single* | | |
| CA125 | 66% | 36% |
| HE4 | 68% | 51% |
| SMRP | 31% | 25% |
| CA72-4 | 25% | 23% |
| Osteopontin | 14% | 4% |

| *Combined with CA125* | | |
|---|---|---|
| CA125 + HE4 | 74% | 68% |
| CA125 + SMRP | 57% | 46% |
| CA125 + CA72-4 | 63% | 51% |
| CA125 + Osteopontin | 66% | 38% |

| *Optimal 3* | | |
|---|---|---|
| CA125 + HE4 + CA72-4 | 70% | 66% |

| *Optimal 4* | | |
|---|---|---|
| CA125 + HE4 + CA72-4 + SMRP | 66% | 66% |

| *All* | | |
|---|---|---|
| CA125 + HE4 + CA72-4 + SMRP + Osteopontin | 66% | 63% |

### Example 2

These are the methods that were used in this example.

Serum and urine samples were obtained pre-operatively following consent. Urine was collected either from catheterization from a voided pre-operative specimen. A whole blood specimen of 30mL and a urine sample of > 10mL were collected. The whole blood was collected into three 10mL Serum Separator Tubes (SST). The SST tubes were centrifuged, separated, and the supernatant frozen within 3 hours of collection at < -80oC. The 10mL urine sample was collected in, or transferred into, an appropriate tube for shipment and storage, and was also frozen on the day of collection at < -80°C. The serum and urine samples were shipped in batches to the Fujirebio Diagnostics laboratory for testing and long term storage. The following serum tumor marker levels were analyzed for CA125, HE4, SMRP, CA72-4, activin, inhibin and Osteopontin as well as urinary SMRP.

Post-operatively, the pathology reports were reviewed for all patients and classified as benign or malignant. The histologic type of malignancy and the specific diagnosis for those with benign disease were also recorded, as was the stage of disease for all patients with ovarian cancer. All tumor markers were compared to the final pathology results. Mean and median values were compared between those with benign disease and those with cancer using t-test and chi-square as appropriate. Subset analysis comparing only those with Stage I cancer to those with benign disease was also performed. Logistic regression models were estimated for each marker individually and all combinations of markers. Cross-validation analysis was performed to obtain the sensitivity at 90, 95% and 98% specificity for each marker individually as well as in combination. Sensitivity analysis was also performed for the Stage I subset.

These are the results from this example.

Two-hundred and forty-four patients with adnexal masses were enrolled. Two women were excluded who did not have serum obtained preoperatively, and seven were excluded who were found to have metastasis from distant primaries cancers. 235 patients were thus eligible for analysis. Pathology results in these women revealed 69 ovarian cancers (14 stage I) and 166 benign ovarian masses. The age distribution in those with and without malignancy is shown in Table 2, and patients with cancer were not surprisingly older than those with benign disease.

The exact diagnoses found in those women with benign disease are shown in Table 3. The most common diagnosis was benign cystadenoma or cystadenofibroma accounting for approximately one third of women with benign disease The histologic and stage distribution of the ovarian cancers is shown in Table 4. Consistent with ovarian cancer epidemiology, the majority of women (71%) had serous tumors and were found to have Stage III disease (67%).

Mean and median values for HE4, SMRP, CA 125, CA72-4, activin, and inhibin all differed significantly between benign masses and cancer. Osteopontin levels were not found to be significantly different. This data is shown in Table 5.

Sensitivity at 90, 95 and 98% specificity was then calculated for all markers individually and for the marker combinations as shown in Table 6. CA 125 and HE4 were the best individual predictors of malignancy. They are not closely correlated with each other, suggesting that they are identifying different subpopulations of disease. When potential marker combinations were compared, CA125 and HE4 in combination had the highest sensitivity of 77% at a set 95% specificity. The inclusion of additional markers did not significantly increase the sensitivity.

Subset analysis comparing those only those with Stage I disease to those with benign disease was then performed. Mean and median values are shown in Table 7. HE4, CA125, and SMRP were significantly different between those with ovarian cancer and those with benign disease. Sensitivities at varying set specificities are shown in Table 8. HE4 is the most sensitive single marker, with a sensitivity of 50% at 95% specificity. The addition of CA125, or any of the other markers, did not significantly improve on the sensitivity of detection of Stage I disease.

**Table 2**

| AgeDistribution inCases andBenigns | | |
|---|---|---|
| Age Group | Group | |
| (years) | Benign | Cancer |
| <30 | 4 (2%) | 2 (3%) |
| 30 - 39 | 19 (12%) | 3 (5%) |
| 40 - 49 | 58 (35%) | 7 (10%) |
| 50 - 59 | 45 (27%) | 13 (19%) |
| 60 - 69 | 26 (16%) | 16 (23%) |
| 70 - 79 | 9 (5%) | 18 (26%) |
| >=80 | 5 (3%) | 9 (13%) |
| Missing | 0 (0%) | 1 (1%) |
| Total | 166 | 69 |

**Table 3**

| Distribution of Diagnoses in Benigns | |
|---|---|
| Diagnosis | N (%) |
| Endometriosis | 23 (13.9%) |
| Cystadenoma | 30 (18.2%) |
| Cystadenofibroma | 15 (9%) |
| Endosalpingiosis | 4 (2.4%) |
| Myoma | 8 (4.8%) |
| Cyst | 10 (6%) |
| Pelvic inflammatory Disease | 3 (2%) |
| Prophylactic | 2 (2%) |
| Other begin condition | 71 (42.8%) |
| **Total** | **166** |

**Table 4**

| Histologyby StageinCancer Cases | | | | | |
|---|---|---|---|---|---|
| Histology | Stage | | | | Total |
| | I | II | III | IV | |
| Serous | 4 | 1 | 38 | 6 | 49(71%) |
| Mucinous | 3 | 0 | 1 | 0 | 4(5.8%) |
| Endometrioid | 3 | 0 | 2 | 0 | 5(7.2%) |
| Clear Cell | 0 | 1 | 1 | 0 | 2(2.9%) |
| MMMT | 0 | 0 | 1 | 0 | 1(1.4%) |
| Mixed | 4 | 0 | 1 | 0 | 5(7.2%) |
| Neuroendocrine | 0 | 0 | 1 | 0 | 1(1.4%) |
| Poorly differentiated | 0 | 1 | 1 | 0 | 2(2.9%) |
| Total | 14(20%) | 3(4%) | 46(67%) | 6(9%) | 69 |

**Table 5**

| | Benign mean(median) | Ovarian Cancer mean (median) | t-test p-value | Chi² p-value |
|---|---|---|---|---|
| HE4 | 50(40) | 531(231) | <0.0001 | <0.001 |
| SMRP | 0.8(0.6) | 4.5(1.9) | <0.0001 | <0.001 |
| CA125 | 67(14) | 627(262) | <0.0001 | <0.001 |
| CA72-4 | 3.5(1.5) | 32.1(3.3) | <0.0001 | <0.001 |
| Urine SMRP | 0.6(0.1) | 2.2(0.4) | 0.0269 | <0.001 |
| Urine CA125 | 5(2.4) | 8.5(4.4) | 0.0297 | <0.001 |
| Activin | 0.7(0.5) | 1.1(1.0) | 0.0003 | 0.001 |
| Inhibin | 45(26) | 25(9) | 0.0045 | 0.005 |
| Osteopontin | 66(37) | 95(53) | 0.2591 | 0.031 |

**Table 6**

| Average from Leave-One-Out Analysis | Benign vs. Ovarian Cancer: Sensitivity at | | | |
|---|---|---|---|---|
| Marker Combination | ROC-AUC(95% CI) | 90% Specificity | 95% Specificity | 98% Specificity |
| CA125 | 82.7% (76.8 - 88.7) | 59.4% | 42.0% | 23.2% |
| HE4 | 90.6% (85.9 - 95.2) | 76.8% | 71.3% | 62.3% |
| SMRP | 82.7% (76.3 - 89.1) | 60.9% | 53.6% | 43.5% |
| CA72-4 | 76.7% (69.8 - 83.6) | 42.0% | 31.1% | 22.8% |
| Osteopontin | 64.5% (57.0 - 72.0) | 22.6% | 7.6% | 5.4% |
| Urine SMRP | 71.0% (62.8 - 79.3) | 36.5% | 32.0% | 25.4% |
| Urine CA125 | 72.6% (65.2 - 79.9) | 30.6% | 15.5% | 3.2% |
| Activin | 69.0% (61.6 - 76.4) | 30.4% | 23.2% | 13.0% |
| Inhibin | 35.2% (27.8 - 42.6) | 7.1% | 0.0% | 0.0% |
| CA125 + HE4 | 91.1% (86.5 - 95.7) | 81.0% | 76.8% | 68.3% |
| CA125 + SMRP | 86.4% (80.8 - 91.9) | 73.9% | 57.7% | 50.7% |
| CA125 + CA72-4 | 85.3% (80.1 - 90.5) | 60.9% | 44.9% | 30.5% |
| CA125 + Osteopontin | 82.5% (75.4 - 88.5) | 58.8% | 41.1% | 23.5% |
| CA125 + Urine SMRP | 79.9% (72.5 - 87.3) | 59.8% | 36.9% | 23.9% |
| CA125 + Urine CA125 | 79.2% (71.6 - 86.9) | 59.9% | 39.7% | 22.4% |
| CA125 + Activin | 80.9% (74.4 - 87.4) | 62.3% | 43.0% | 25.9% |
| CA125 + Inhibin | 80.9% (74.2 - 87.5) | 62.8% | 41.9% | 23.2% |
| HE4 + SMRP | 91.5% (87.2 - 95.7) | 78.5% | 71.0% | 63.8% |
| HE4 + CA72-4 | 90.7% (86.1 - 95.3) | 76.8% | 68.1% | 65.2% |
| HE4 + Osteopontin | 90.5% (85.7 - 95.3) | 78.0% | 73.5% | 62.8% |
| HE4 + Urine SMRP | 89.6% (84.5 - 94.7) | 74.6% | 66.0% | 58.7% |
| HE4 + Urine CA125 | 89.5% (84.4 - 94.6) | 74.6% | 68.1% | 60.3% |
| HE4 + Activin | 90.8% (86.3 - 95.3) | 76.8% | 71.1% | 62.3% |
| HE4 + Inhibin | 90.4% (85.8 - 94.9) | 79.6% | 73.8% | 62.5% |
| CA125 + HE4 + SMRP | 91.1% (86.4 - 95.7) | 79.7% | 72.5% | 69.6% |
| CA125 + HE4 + CA72-4 | 91.1% (86.5 - 95.7) | 81.2% | 77.1% | 68.2% |
| CA125 + HE4 + Osteopontin | 91.2% (86.5 - 95.9) | 80.7% | 77.9% | 70.6% |
| CA125 + HE4 + Urine SMRP | 90.3% (85.2 - 95.4) | 77.9% | 71.5% | 68.2% |
| CA125 + HE4 + Urine CA125 | 90.0% (84.7 - 95.3) | 77.8% | 72.8% | 66.7% |
| CA125 + HE4 + Activin | 91.1% (86.4 - 95.7) | 81.2% | 73.9% | 68.2% |
| CA125 + HE4 + Inhibin | 91.3% (86.8 - 95.7) | 79.8% | 75.5% | 66.8% |
| CA125 + HE4 + SMRP + CA72-4 | 91.2% (86.6 - 95.8) | 81.0% | 72.5% | 69.6% |
| CA125 + HE4 + CA72-4 + Urine SMRP | 90.3% (85.2 - 95.4) | 77.9% | 71.6% | 67.4% |
| CA125 + HE4 + CA72-4 + SMRP + Osteopontin | 91.3% (86.6 - 95.9) | 80.5% | 74.2% | 70.6% |

**Table 7**

| | Benign mean(median) | Stage 1 Ovarian Cancer mean(median) | t-test p-value | Chi² p-value |
|---|---|---|---|---|
| HE4 | 50(40) | 207(98) | <0.0001 | 0.012 |
| SMRP | 0.8(0.6) | 1.2(0.8) | 0.068 | 0.651 |
| CA125 | 67(14) | 847(68) | 0.0005 | 0.051 |
| CA72-4 | 3.5(1.5) | 66(2.3) | <0.0001 | 0.015 |
| Osteopontin | 66(37) | 158(50) | 0.1008 | 0.404 |

**Table 8**

| Average from Leave-One-Out Analysis | Benign vs. Ovarian Cancer Stage 1: Sensitivity at | | | |
|---|---|---|---|---|
| Marker Combination | ROC-AUC(95% CI) | 90% Specificity | 95% Specificity | 98% Specificity |
| CA125 | 67.6% (53.4 - 81.8) | 19.7% | 7.7% | 7.1% |
| HE4 | 77.8% (63.2 - 92.3) | 50.0% | 49.2% | 28.6% |
| SMRP | 66.2% (51.6 - 80.7) | 28.6% | 28.5% | 14.3% |
| CA72-4 | 74.8% (61.7 - 87.8) | 34.8% | 34.7% | 21.4% |
| Osteopontin | 57.6% (42.9 - 72.2) | 7.6% | 7.6% | 7.1% |
| CA125 + HE4 | 76.6% (61.1 - 92.0) | 50.0% | 42.7% | 35.7% |
| CA125 + SMRP | 72.6% (59.9 - 85.4) | 35.7% | 28.5% | 21.4% |
| CA125 + CA72-4 | 70.7% (57.2 - 84.2) | 28.1% | 21.4% | 21.4% |
| CA125 + Osteopontin | 63.9% (51.8 - 76.1) | 21.3% | 14.9% | 14.2% |
| CA125 + HE4 + CA72-4 | 76.9% (61.6 - 92.1) | 50.0% | 35.8% | 35.7% |
| CA125 + HE4 + SMRP + CA72-4 | 76.9% (62.3 - 91.6) | 50.6% | 35.7% | 35.7% |
| CA125 + HE4 + CA72-4 + SMRP + Osteopontin | 77.0% (62.4 - 91.6) | 50.4% | 35.8% | 35.6% |

### Example 3

These are the methods used in this example.

Data from two separate IRB approved prospective trials from two institutions were collected. After obtaining informed consent, serum samples were obtained preoperatively from women undergoing surgery for an adnexal mass and analyzed for levels of CA125, SMRP, HE4 and CA72-4. All pathology results were compared to the tumor markers. Sensitivities at set specificities of 90, 95 and 98% were determined using logistic regression for each marker individually and all combinations of 2, 3, and 4 markers.

These are the results from this example.

448 samples were analyzed. There were 267 benign cases and 181 ovarian cancers (27 stage I, 20 stage II, 115 stage III and 19 stage IV). Median values for HE4, SMRP, CA125 and CA72-4 all differed significantly between benign masses and cancer (p<0.001). In the differentiation of benign masses and stage I malignancies, the addition of HE4 to CA125 increased the sensitivity by 22.2% at a specificity of 90%. The combination of HE4 and CA125 for all stages was superior to HE4 or CA125 alone (p<0.003). The results are hereby summarized in Table 9.

**Table 9**

| Panels | Sensitivity for Benign vs. all Stages | | |
|---|---|---|---|
| | 90% Specificity | 95% Specificity | 98% Specificity |
| *Single Markers* | | | |
| CA125 | 65.7 | 58.0 | 32.6 |
| HE4 | 69.6 | 58.0 | 45.9 |
| SMRP | 54.1 | 45.3 | 40.3 |
| CA72-4 | 52.2 | 35.6 | 32.2 |

| *Optimal Combinations* | | | |
|---|---|---|---|
| CA125 + HE4 | 74.0 | 68.5 | 60.8 |
| CA125 + HE4 + SMRP | 75.1 | 70.2 | 64.6 |
| CA125 + HE4 + CA72-4 | 75.6 | 70.0 | 63.3 |
| CA125 + HE4 + CA72-4 + SMRP | 77.2 | 70.6 | 66.7 |

While this subject matter has been disclosed with reference to specific embodiments, it is apparent that other embodiments and variations can be devised by others skilled in the art.

## Claims

1. A method of assessing whether a patient is afflicted with stage I ovarian cancer, the method comprising
assessing at least two markers, including both the HE4 marker and another marker selected from the group consisting of SMRP, CA125, and CA72-4, in a biological fluid sample obtained from the patient,
wherein elevated levels of the markers are correlated with increased likelihood that the patient is afflicted with stage I ovarian cancer.

2. The method of claim 1 wherein the patient exhibits a pelvic mass.

3. A method of differentiating whether a pelvic mass in a patient is benign or a stage I ovarian cancer, the method comprising assessing at least two markers, including both the HE4 marker and another marker selected from the group consisting of SMRP, CA125, and CA72-4, in a biological fluid sample obtained from the patient, wherein elevated levels of the markers are correlated with increased likelihood that the pelvic mass is a stage I ovarian cancer.

4. A method of assessing the response of a patient afflicted with stage I ovarian cancer to a treatment, the method comprising assessing at least two markers, including both the HE4 marker and another marker selected from the group consisting of SMRP, CA125, and CA72-4, in biological fluid samples obtained from the patient at different times during treatment, wherein decreased levels of the markers at the later time indicate that the patient is responding to the treatment.

5. A method of assessing the likelihood that a patient will develop a stage I ovarian cancer, the method comprising assessing at least two markers, including both the HE4 marker and another marker selected from the group consisting of SMRP, CA125, and CA72-4, in a biological fluid sample obtained from the patient, wherein elevated levels of the markers are correlated with increased likelihood that the patient will develop a stage I ovarian cancer.

6. The method of any preceding claim, comprising assessing the HE4 and CA125 markers.

7. The method of any preceding claim, comprising assessing the HE4 and SMRP markers.

8. The method of claim 4, wherein the treatment is intraperitoneal chemotherapy.

9. The method of claim 4, wherein the treatment comprises administration to the patient of an antibody that binds specifically with CA125.

10. The method of claim 6 as dependent on claim 5, wherein the patient exhibits normal levels of the CA125 marker and an elevated level of the HE4 marker is correlated with increased likelihood that the patient will develop a stage I ovarian cancer.

## Patentansprüche

1. Ein Verfahren zum Beurteilen, ob ein Patient von Eierstockkrebs des Stadiums I betroffen ist, wobei das Verfahren Folgendes beinhaltet:
Beurteilen von mindestens zwei Markern, die sowohl den HE4-Marker als auch einen anderen Marker umfassen, der aus der Gruppe ausgewählt ist, die aus SMRP, CA125 und CA72-4 besteht, in einer von dem Patienten erhaltenen biologischen Flüssigkeitsprobe,
wobei erhöhte Pegel der Marker mit einer vergrößerten Wahrscheinlichkeit korreliert sind, dass der Patient von Eierstockkrebs des Stadiums I betroffen ist.

2. Verfahren gemäß Anspruch 1, wobei der Patient eine Beckenmasse aufweist.

3. Verfahren zum Unterscheiden, ob eine Beckenmasse bei einem Patient gutartig oder ein Eierstockkrebs des Stadiums I ist, wobei das Verfahren das Beurteilen von mindestens zwei Markern, die sowohl den HE4-Marker als auch einen anderen Marker umfassen, der aus der Gruppe ausgewählt ist, die aus SMRP, CA125 und CA72-4 besteht, in einer von dem Patienten erhaltenen biologischen Flüssigkeitsprobe beinhaltet, wobei erhöhte Pegel der Marker mit einer vergrößerten Wahrscheinlichkeit korreliert sind, dass die Beckenmasse ein Eierstockkrebs des Stadiums I ist.

4. Verfahren zum Beurteilen der Reaktion eines Patienten, der von Eierstockkrebs des Stadiums I betroffen ist, auf eine Behandlung, wobei das Verfahren das Beurteilen von mindestens zwei Markern, die sowohl den HE4-Marker als auch einen anderen Marker umfassen, der aus der Gruppe ausgewählt ist, die aus SMRP, CA125 und CA72-4 besteht, in von dem Patienten zu unterschiedlichen Zeitpunkten während der Behandlung erhaltenen biologischen Flüssigkeitproben, wobei verminderte Pegel der Marker zum späteren Zeitpunkt anzeigen, dass der Patient auf die Behandlung reagiert.

5. Verfahren zum Beurteilen der Wahrscheinlichkeit, dass ein Patient einen Eierstockkrebs des Stadiums I entwickeln wird, wobei das Verfahren das Beurteilen von mindestens zwei Markern, die sowohl den HE4-Marker als auch einen anderen Marker umfassen, der aus der Gruppe ausgewählt ist, die aus SMRP, CA125 und CA72-4 besteht, in einer von dem Patienten erhaltenen biologischen Flüssigkeitsprobe beinhaltet, wobei erhöhte Pegel der Marker mit einer vergrößerten Wahrscheinlichkeit korreliert sind, dass einen Eierstockkrebs des Stadiums I entwickeln wird.

6. Verfahren gemäß einem vorherigen Anspruch, das das Beurteilen der HE4- und CA125-Marker beinhaltet.

7. Verfahren gemäß einem vorherigen Anspruch, das das Beurteilen der HE4- und SMRP-Marker beinhaltet.

8. Verfahren gemäß Anspruch 4, wobei die Behandlung intraperitoneale Chemotherapie ist.

9. Verfahren gemäß Anspruch 4, wobei die Behandlung die Verabreichung an den Patienten eines Antikörpers beinhaltet, der spezifisch an CA125 bindet.

10. Verfahren gemäß Anspruch 6 in Abhängigkeit von Anspruch 5, wobei der Patient normale Pegel des CA125-Markers aufweist und ein erhöhter Pegel des HE4-Markers mit einer vergrößerten Wahrscheinlichkeit korreliert ist, dass der Patient einen Eierstockkrebs des Stadiums I entwickeln wird.

## Revendications

1. Procédé permettant d'évaluer si un patient souffre du cancer ovarien au stade I, le procédé comprenant
l'évaluation d'au moins deux marqueurs, y compris à la fois le marqueur HE4 et un autre marqueur choisi dans le groupe constitué de SMRP, de CA125 et de CA72-4, dans un échantillon de fluide biologique provenant du patient,
dans lequel des niveaux élevés des marqueurs sont corrélés à une probabilité accrue que le patient souffre du cancer ovarien au stade I.

2. Procédé selon la revendication 1 dans lequel le patient présent une masse pelvienne.

3. Procédé permettant de différencier si une masse pelvienne chez un patient est bégnine ou est un cancer ovarien au stade I, le procédé comprenant l'évaluation d'au moins deux marqueurs, y compris à la fois le marqueur HE4 et un autre marqueur choisi dans le groupe constitué de SMRP, de CA125 et de CA72-4, dans un échantillon de fluide biologique provenant du patient, dans lequel des niveaux élevés des marqueurs sont corrélés à une probabilité accrue que la masse pelvienne est un cancer ovarien au stade I.

4. Procédé permettant d'évaluer la réponse d'un patient qui souffre du cancer ovarien au stade I à un traitement, le procédé comprenant l'évaluation d'au moins deux marqueurs, y compris à la fois le marqueur HE4 et un autre marqueur choisi dans le groupe constitué de SMRP, de CA125 et de CA72-4, dans des échantillons de fluide biologique provenant du patient à différents moments du traitement, dans lequel des niveaux réduits des marqueurs à l'instant ultérieur indiquent que le patient répond au traitement.

5. Procédé permettant d'évaluer la probabilité qu'un patient va développer un cancer ovarien au stade I, le procédé comprenant l'évaluation d'au moins deux marqueurs, y compris à la fois le marqueur HE4 et un autre marqueur choisi dans le groupe constitué de SMRP, de CA125 et de CA72-4, dans un échantillon de fluide biologique provenant du patient, dans lequel des niveaux élevés des marqueurs sont corrélés à une probabilité accrue que le patient va développer un cancer ovarien au stade I.

6. Procédé selon l'une quelconque des revendications précédentes, comprenant l'évaluation des marqueurs HE4 et CA125.

7. Procédé selon l'une quelconque des revendications précédentes, comprenant l'évaluation des marqueurs HE4 et SMRP.

8. Procédé selon la revendication 4, dans lequel le traitement est une chimiothérapie intrapéritonéale.

9. Procédé selon la revendication 4, dans lequel le traitement comprises l'administration au patient d'un anticorps qui se lie spécifiquement à CA125.

10. Procédé selon la revendication 6 telle que dépendante de la revendication 5, dans lequel le patient présente des niveaux normaux du marqueur CA125 et un niveau élevé du marqueur HE4 est corrélé à une probabilité accrue que le patient va développer un cancer ovarien au stade I.
